# EUROPÄISCHE PATENTSCHRIFT

(11) **EP 3 095 866 B1**
(45) Veröffentlichungstag und Bekanntmachung des Hinweises auf die Patenterteilung: **08.05.2019**
(21) Anmeldenummer: 15168512.0
(22) Anmeldetag: 20.05.2015
(51) Int. Cl.: C12N 15/113, A61P 31/16, A61K 31/711

(54) **MITTEL ZUR PROPHYLAXE UND THERAPIE VON VIRUSINFEKTIONEN**
AGENT FOR THE PROPHYLAXIS AND THERAPY OF VIRAL INFECTIONS
MOYEN DE PROPHYLAXIE ET THÉRAPIE D'INFECTIONS VIRALES

(43) Veröffentlichungstag der Anmeldung: 23.11.2016
(73) Patentinhaber: Secarna Pharmaceuticals GmbH & Co. KG, 35037 Marburg (DE)
(72) Erfinder: Potaczek, Daniel, 35039 Marburg (PL); Garn, Holger, 35037 Marburg (DE); Renz, Harald, 35043 Marburg (DE)
(74) Vertreter: V.O.

(56) Entgegenhaltungen:
- WO-A2-2004/042046
- WO-A2-2007/030576
- SCHUBERT S ET AL: "RNA cleaving '10-23' DNAzymes with enhanced stability and activity", NUCLEIC ACIDS RESEARCH, INFORMATION RETRIEVAL LTD, Bd. 31, Nr. 20, 15. Oktober 2003 (2003-10-15), Seiten 5982-5992, XP002272988, ISSN: 0305-1048, DOI: 10.1093/NAR/GKG791
- SCHUBERT S ET AL: "Gaining Target Access for Deoxyribozymes", JOURNAL OF MOLECULAR BIOLOGY, ACADEMIC PRESS, UNITED KINGDOM, Bd. 339, Nr. 2, 28. Mai 2004 (2004-05-28), Seiten 355-363, XP004506522, ISSN: 0022-2836, DOI: 10.1016/J.JMB.2004.03.064

## Beschreibung

Die vorliegende Erfindung betrifft ein Mittel zur Prophylaxe und Behandlung von Virusinfektionen, die durch Picornaviren insbesondere durch Rhinoviren verursacht bzw. hervorgerufen werden.

### Picornaviren

Picornaviren sind eine spezielle Gruppe von RNA-Viren, die bei Menschen und verschiedenen anderen Säugetieren Infektionen und Krankheiten auslösen. Das virale Genom der Picornaviren umfasst eine einzelsträngige RNA mit positiver Polarität. Zwischen zwei nichtcodierenden Bereichen am 3'- und 5'-Ende liegt ein einziger offener Leserahmen für ein virales Vorläuferpolyprotein, das während der Translation in einzelne Virusproteine gespalten wird. Am 3'- Ende liegt der für positivsträngige Viren typische Poly-A-Schwanz. Am 5'- Ende liegt vor dem Startcodon ein Bereich, in dem die RNA durch zahlreiche komplementäre Basenpaarungen eine komplexe Sekundärstruktur aufweist. Funktionell entspricht dieser Abschnitt einer IRES (internal ribosomal entry site) zur Initiation der Translation an den Ribosomen.

Zu den Picornaviridae zählt man:
- Coxsackieviren
- Echoviren
- Enteroviren
- Polioviren
- Rhinoviren

Infektionen mit diesen Viren verursachen eine Reihe klinisch relevanter Erkrankungen, wie z.B. Aseptische Meningitis, Herpangina (auch Zahorsky-Krankheit), Hand-Fuß-Mund-Krankheit, Hämorrhagische Konjunktivitis, Erkrankungen der Atemwege (z.B. Sommergrippe, Pharyngitis, Pneumonie) und Erkrankungen innerer Organe (z. B. Perikarditis, Myokarditis, Pleurodynie).

### Diagnostik

Aufgrund der Vielfalt der möglichen in Frage kommenden Erreger erfolgt die sichere Diagnostik in einer biologischen Probe eines Menschen oder Säugetieres immer noch durch den direkten Erregernachweis mittels Virusanzucht in Zellkultur mit anschließender Typisierung (Neutralisationstest) bzw. Nachweis des Virusgenoms mit molekularen Methoden (Nukleinsäure Amplifikationstechniken, wie z. B. RT-PCR). Dieser direkte Nachweis in einer biologischen Probe wie Blut, Stuhl, Liquor oder Rachenspülwasser ist sehr aufwendig und teuer.

### Therapie

Auch eine spezifische medikamentöse Therapie der Virusinfektion ist derzeit nicht möglich. Die Therapie erfolgt ausschließlich symptomatisch und richtet sich nach dem betroffenen Organsystem.

### Prophylaxe

Es gibt eine Reihe von prophylaktischen Hygienemaßnahmen, um eine Virusinfektion zu verhindern. Der wichtigste Schutz vor Infektionen und Erkrankungen ist die Impfung. Gegen Polioviren steht ein solcher Impfschutz zur Verfügung, für Rhinoviren und andere Viren aus der Gruppe der Picornaviridae stehen dagegen keine oder kaum wirksame Impfstoffe zur Verfügung.

### Rhinoviren

Zu der Gruppe der Rhinoviren gehören ca. 157 verschiedene Typen, die aufgrund der Homologie im Genom in drei Gruppen A, B und C eingeteilt werden. Rhinoviren der Gruppe A und B binden meist an den zellulären ICAM-1- Rezeptor oder an den LDL-Rezeptor.

Die viralen Proteine werden als Polyprotein hergestellt und danach durch Proteasen gespalten. Die im Virion befindlichen strukturellen Proteine werden im 5'-Bereich der RNA codiert, die nicht-strukturellen Proteine im 3'-Bereich. Am 5'-Ende ist ein nichttranslatierter Bereich (5'-UTR) dem sich die P1-Region, die P2-Region und die P3-Region anschließen, die jeweils für Kapsidproteine (VP1 bis VP4), Protease und RNA-Polymerase kodieren. Es folgt ein weiterer nichttranslatierter Bereich (3'-UTR) und ein Poly-A-Schwanz. Die Kapsidproteine (VP1 bis VP4) dienen zur Verpackung des Genoms und auch als Rezeptor für die Anheftung an eine Wirtszelle. VP1-3 werden als Oberflächenproteine von Antikörpern des Wirtsorganismus erkannt.

Rhinoviren sind weltweit verbreitet und auf den Menschen begrenzt. Sie bevorzugen zu ihrer Vermehrung Temperaturen von 3 °C bis 33 °C, vermehren sich allerdings auch bei höheren Temperaturen, insbesondere wenn sich die Infektionen nicht nur auf den Schleimhautbereich sondern auch auf den Atemwegs- und Lungenbereich erstreckt.

Die Rhinitis acuta (Erkältungsschnupfen) ist die häufigste durch Rhinoviren hervorgerufene Infektionskrankheit des Menschen, die zu Schnupfen, Asthma oder Steigerung der Atemwegsempfindlichkeit führt. Bei bis zu 40 Prozent aller akuten Asthmaanfälle oder COPD (chronisch obstruktive Lungenerkrankung) Exazerbationen sind virale Infektionen der oberen Atemwege beteiligt. Die Viren schädigen die Epithelschicht der Atemwege, lösen eine Entzündung aus und steigern die nervale Empfindlichkeit der Atemwegszellen. Bei Asthmatikern führt dies zu schweren Konsequenzen, aber auch gesunde Personen sind betroffen. Daher leiden manche Menschen nach einer »Erkältung« unter einem persistierenden Husten.

Während Infektionen mit Rhinoviren bei gesunden Menschen gewöhnlich eine harmlose und rasch abklingende Erkältung hervorrufen, können die Infektionen bei Asthmatikern und Menschen, die bereits eine Empfindlichkeit der Atemwegszellen aufweisen, sehr stark werden und lebensbedrohliche Atemnot, Brustenge und Kurzatmigkeit auslösen.

Britische Wissenschaftler zeigten kürzlich, dass Infektionen mit Rhinoviren in Lungenepithelzellen des Menschen die verstärkte Bildung von Cytokinen, insbesondere Interleukin (IL-25) auslösen. Dies führt zu einer Signalkaskade wie bei einer allergischen Reaktion und letztlich zu einer ungewöhnlich starken Entzündungsreaktion.

In diesem Zusammenhang ist auch bekannt, dass die Infektion mit Rhinoviren bei Kindern zu einer Empfindlichkeit der Atemwegszellen und zu einer höheren Inzidenz für die Ausbildung von Asthma im Erwachsenenalter führen.

### Prophylaxe

Es ist die vorherrschende wissenschaftliche Meinung, dass eine Prophylaxe vor einer Infektion mit Rhinoviren kaum möglich ist, ebenso wenig eine kausale Therapie, sodass es dem Immunsystem des Menschen zukommt, dagegen anzukommen. Allerdings ist gerade das Immunsystem von Neugeborenen, Patienten mit natürlicher oder Medikamenten bedingter Immunsuppression extrem geschwächt, so dass eine Prophylaxe vor einer Infektion mit Picornaviren insbesondere Rhinoviren wünschenswert ist.

### Therapie

Eine kausale Therapie gibt es nicht, zwangsläufig bedeutet dies für den Menschen das Durchleiden der Krankheitsstadien. Ihm stehen nur Mittel für eine symptomatische Behandlung der Begleiterscheinungen Schnupfen und Kopfschmerzen zur Verfügung. Bekannte symptomatische Maßnahmen sind körperliche Schonung, Inhalationen, Einreibungen mit ätherischen Ölen und gesunde Ernährung. Hinzu kommen diverse nasale Arzneiformen mit Wirkstoffen, die die Nasenschleimhaut abschwellen oder pflegen sollen. Der Fachmann kennt Nasensprays mit den Wirkstoffen Tramazolin und Xylometazolin, die die Atemwege kurzzeitig befreien. Nasensprays mit dem Wirkstoff Oxymetazolin haben eine direkte antivirale Wirkung durch Verhinderung der Expression von ICAM-1, dem Rezeptor für die Rhinoviren. Allerdings wirken diese nur gegen das Phänomen Schnupfen und nicht bei den schweren Virusinfektionen wie Asthma oder COPD (chronisch obstruktive Lungenerkrankung). Weiterhin werden Lutschtabletten gegen das Kratzen im Hals sowie Brausetabletten, Säfte oder Tropfen mit Wirkstoffen, die das Abhusten von zähem Schleim erleichtern oder Husten lindern sollen, eingesetzt, zuweilen werden dem Patienten kurzzeitig auch Analgetika und Antipyretika empfohlen. Vielfach kommt es in Folge einer Virusinfektion auch zu einer bakteriellen Superinfektion und damit auch zu Lungenentzündung. Diese werden dann mit verschiedenen Antibiotika behandelt, wobei die Auswirkungen bei vorliegenden Resistenzen allgemein bekannt sind. Bei der Behandlung von Asthma oder COPD ist sogar ein Wechsel von oraler zu systemischer Behandlung erforderlich, wobei Glucocorticoide eingesetzt werden müssen und die Behandlung im Krankenhaus teuer und aufwendig ist.

### Impfung

Eine spezifische Impfung gegen Rhinoviren gibt es bislang nicht. In den 1960er Jahren wurden verschiedene Impfstoffe erzeugt, die jedoch nur einen spezifischen Impfschutz, gegen das oder die speziellen Rhinoviren der Saison bzw. der Gegend erzeugen konnten.

Allerdings bewirkt auch eine Rhinovirus-Infektion im Körper nur eine Immunität gegen den bestimmten Infektionstyp. Eine charakteristische Eigenschaft von RNA-Viren allgemein ist deren verstärkte Mutationsrate und die damit bedingte Flexibilität. So sind beispielsweise die Kapsidproteine von Rhinoviren auf Proteinebene sehr variabel, was die Bildung einer übergreifenden Immunität sehr erschwert und auch die Herstellung eines Impfstoffes, der gegen mehr als einen Rhinovirustyp wirksam ist bislang unmöglich machte. Derzeit gibt es keine langfristig wirksamen spezifischen Medikamente oder Impfstoffe. Zwar wird im Stand der Technik der Wirkstoff Pleconaril, ein Kapsidblocker erwähnt, der mit dem Kapsidprotein VP1 interagiert, ein Handelspräparat ist jedoch nicht erhältlich oder zugelassen.

### DNAzyme

DNAzyme sind katalytisch aktive Einzelstrang DNA-Moleküle.

Ein bekannte DNAzym Familie sind die "10-23"-DNAzyme, die die Ziel-Sequenzen von RNA-Molekülen spezifisch erkennen, komplementär binden und durch katalytische Aktivität spalten, wobei die Aktivität des gespaltenen RNA-Moleküls verloren geht oder herabgesetzt wird. Dies ist therapeutisch ein vielversprechender Ansatz für die Behandlung von Krankheiten des menschlichen oder tierischen Körpers, die durch verstärkte Expression von RNA Molekülen verursacht werden. Voraussetzung ist jedoch, dass die Zielstruktur bzw. die Zielsequenz für die Bindung ausreichend identifiziert und zugänglich ist und die DNAzyme sowohl gute Bindeeigenschaft als auch katalytische Aktivität entfalten können.

10-23-DNAzyme weisen eine katalytische Domäne von 15 Nukleotiden auf, welche von zwei Substratbindungsdomänen (I und II) flankiert wird. Die Bindung an das RNA-Substrat erfolgt durch Basenpaarung gemäß den Watson-Crick Regeln über die Substratbindungsdomänen I und II.

### Stand der Technik

Es ist bekannt Antisense-Strategie zu nutzen, um krankheitsauslösende RNA-Moleküle im menschlichen oder tierischen Körper zu binden und zu blockieren.

Der Stand der Technik kennt "10-23"-DNAzyme, die hochspezifisch RNA- Zielstrukturen erkennen, binden und schneiden. Damit wird die Zielstruktur inaktiviert, gehemmt bzw. blockiert, so dass sie ihre krankheitsauslösende Funktion im Organismus nicht mehr erfüllen kann. Ausgewählte DNAzyme sind für den medizinischen Einsatz als Mittel beispielsweise zur Hemmung der Virus-Replikation beschrieben. Diese DNAzyme sind jedoch sehr spezifisch gegen konservierte Regionen spezifischer RNA-Viren eines Typs gerichtet und sind somit nicht in der Lager eine Gruppe von mehreren Virustypen oder sich strak verändernden Virustypen abzudecken. Sie weisen keine breite Anwendbarkeit bei mehreren Virustypen oder sich stark verändernden Virustypen auf, wie es die Picornaviren darstellen. Die DE103 22 662A1 offenbart ebenfalls vom "10-23" DNAzyme abgewandelte DNAzyme. Diese erkennen und binden an einer Zielsequenz IRES von Rhinovirus vom Serotyp HRV14.

Nachteilig ist, dass diese Zielsequenz weniger konserviert ist und einer hohen Mutationsrate unterliegt. Daher ist die Wirksamkeit der DNAzyme nur auf Rhinoviren vom Serotyp HRV14 beschränkt und nicht für alle Serotypen der Rhinoviren oder alle Serotypen der Picornaviren allgemein wirksam.

In US 20110091501 werden verbesserte Rhinovirus-Vektoren und deren Sequenzen beschrieben, wie sie als Transportvehikel für Immunogene z.B. InfluenzaVirus-Immunogene in der Therapie verwendet und eingesetzt werden. Der offenbarte Vektor ist mit den Nukleotidsequenzen des Rhinovirus vom Serotyp HRV14 offenbart. Er wird aber nicht als Antisense Molekül eingesetzt, um Rhinovirus mRNA zu binden.

In US20130309238 wird eine Möglichkeit offenbart um die durch Rhinovirusassoziierte Entzündungsreaktionen und Asthma durch antisense Blockade des Midline-1 Reaktionsweges zu realisieren. Hier wird zwar ein catalytic *antisense* construct erwähnt, jedoch keine konkreten DNAzyme gegen Rhinoviren von speziellen oder möglichst vielen Serotypen.

Keine der gefundenen Druckschriften aus dem Stand der Technik offenbart spezifische DNAzyme die gegen eine Vielzahl von Virusinfektionen, die durch Picornaviren insbesondere Rhinoviren ausgelöst werden, einsetzbar sind.

### Aufgabe

Aufgabe der vorliegenden Erfindung ist es die Nachteile im Stand der Technik zu beseitigen und ein Mittel zur Prophylaxe und Therapie von Virusinfektionen bereitzustellen, die durch Picornaviren insbesondere Rhinoviren ausgelöst werden und das gegen eine Vielzahl von Serotypen wirksam ist.

### Lösung der Aufgabe

Diese Aufgabe wird erfindungsgemäß gelöst durch ein Mittel zur Prophylaxe und Behandlung von Virusinfektionen die durch Picornaviren verursacht werden, dadurch gekennzeichnet, dass es mindestens ein DNAzym vom Typ 10-23 ausgewählt aus der Gruppe bestehend aus Seq ID No. 2-7, 15, 16, 23-29, 34-38, 44 und 49-51 aufweist, wobei das DNAzym gemäß Seq ID Nr. 2-7, 15, 16 (dpp-X1) mindestens 8 Nukleotide für eine Substratbindungsdomäne I und mindestens 8 Nukleotide für eine Substratbindungsdomäne II aufweist, das DNAzym gemäß Seq ID Nr. 23-29, 34-38 (dpp-X2) mindestens 6 Nukleotide für die Substratbindungsdomäne I und mindestens 7 Nukleotide für Substratbindungsdomäne II aufweist und/oder das DNAzym gemäß Seq ID Nr. 44, 49-51 (dpp-j-9) mindestens 6 Nukleotide für die Substratbindungsdomäne I und mindestens 10 Nukleotide für Substratbindungsdomäne II aufweist.

### 1. Zielsequenz zur Bindung der erfindungsgemäßen DNAzyme

Überraschenderweise wurde in einem mittels Bioinformatik ausgewerteten Alignment sämtlicher verfügbarer Genomsequenzen von Picornaviren eine sehr große Homologie bzw. Sequenzidentität im 5'-UTR Bereich des Virusgenoms identifiziert. Diese Region ist sehr konserviert und weist eine geringe Mutationsrate innerhalb der Viren auf.

Genauere Auswertungen sämtlicher verfügbarer Genomsequenzen von Rhinoviren (nach Mclntyre et al. J Gen Virol 2012) zeigen ebenfalls in diesem 5'-UTR Bereich eine sehr große Homologie bzw. Sequenzidentität.

Dieser homologe 5'-UTR Bereich Seq. ID 1 stellte somit eine gute Ausgangsposition dar, um DNAzyme bereitzustellen, die für mRNA möglichst vieler Picornaviren und möglichst vieler Serotypen von Rhinoviren komplementär und spezifisch sind. Gegen diese mRNA aus dem 5'-UTR Bereich des Virusgenoms richten sich die erfindungsgemäßen DNAzyme. Eine Spaltung dieser mRNA durch mindestens eines der erfindungsgemäßen DNAzyme hemmt die Replikation vieler Picornaviren und vieler Serotypen von Rhinoviren und wirkt damit dem Krankheitsausbruch entgegen.

Die erfindungsgemäßen DNAzyme sind genau gegen diese Zielsequenz gerichtet. Sie stellen somit ein wirksames Mittel zur Prophylaxe und Therapie von Virusinfektionen dar, die durch Picornaviren insbesondere Rhinoviren ausgelöst werden. Sie sind gegen eine Vielzahl von Serotypen wirksam.

Insbesondere weisen die erfindungsgemäßen DNAzyme ausgewählt aus der Gruppe dpp-X1, dpp-X2 und dpp-j-9 (Seq. ID 2-7, 15, 16, 23-29, 34-38, 44 und 49-51) unerwartet positive Eigenschaften auf hinsichtlich
- Bindung an Zielsequenz im 5'-UTR Bereich des Virusgenoms
- Starke enzymatische Spaltungsaktivität an Zielsequenz im 5'-UTR Bereich
- Starke Inaktivierung der Virus RNA bei verschiedenen Serotypen von Rhinoviren und anderen Picornaviren

Die Spaltung der erfindungsgemäßen DNAzyme wird an allen verfügbaren Genomsequenzen von Picornaviren insbesondere Rhinoviren (nach Mclntyre et al. J Gen Virol 2012 ermittelt.

Es wird eine 97,4 % Homologie und damit Spaltung an allen HRV-A Stämmen ermittelt. Es wird eine 100 % Homologie und damit Spaltung an allen HRV-B Stämmen ermittelt. Es wird eine 98,08 % Homologie und damit Spaltung an allen HRV-C Stämmen ermittelt.

### 2. DNAzyme

### Aufbau der erfindungsgemäßen DNAzyme

Die erfindungsgemäßen DNAzyme ausgewählt aus der Gruppe dpp-X1, dpp-X2 und dpp-j-9 (Seq. ID 2-7, 15, 16, 23-29, 34-38, 44 und 49-51) umfassen jeweils eine zentrale katalytische Domäne von 15 Desoxyribonukleinsäuren mit der Sequenz **GGCTAGCTACAACGA,** welche von zwei Substratbindungsdomänen I und II flankiert wird. Die beiden Substratbindungsdomänen I und II sind wichtig für die katalytische Aktivität des DNAzyms bei der Spaltung der Zielsequenz durch De-Esterifikation. Sie erkennen die spezifische Zielsequenz im 5'-UTR Bereich des Virusgenoms und binden über Watson-Crick Basenpaarung sehr spezifisch.

Die erfindungsgemäßen DNAzyme erkennen konservierte RNA Bereiche des Genoms der Picornaviren insbesondere der Rhinoviren, binden diese Bereiche komplementär und schneiden diese. Damit wird die RNA der Picornaviren insbesondere der Rhinoviren inaktiviert, gehemmt oder blockiert, so dass sie ihre Replikation und damit die krankheitsauslösende Wirkung im Organismus nicht mehr entfalten können. Die erfindungsgemäßen DNAzyme stellen ein wirksames Mittel für den medizinischen Einsatz zur Hemmung der Virus-Replikation der Picornaviren insbesondere der Rhinoviren dar. Der medizinische Einsatz zur Prophylaxe und Behandlung erfolgt am Menschen und/oder Säugetier.

Insbesondere weisen die erfindungsgemäßen DNAzyme ausgewählt aus der Gruppe dpp-X1, dpp-X2 und dpp-j-9 (Seq. ID 2-7, 15, 16, 23-29, 34-38, 44 und 49-51) unerwartet positive Eigenschaften auf hinsichtlich
- Bindung an Zielsequenz im 5'-UTR Bereich des Virusgenoms
- Starke enzymatische Spaltungsaktivität an Zielsequenz im 5'-UTR Bereich
- Starke Inaktivierung der Virus RNA bei verschiedenen Serotypen von Rhinoviren und anderen Picornaviren

Die Länge und Sequenz der Substratbindungsdomänen I und II ist entscheidend für die Spaltungseigenschaften und für die katalytische Aktivität des DNAzymes. Die Substratbindungsdomänen I und II sind entweder gleich oder verschieden lang. Es wurden zahlreiche experimentelle Serien durchgeführt, um für die erfindungsgemäßen DNAzyme ausgewählt aus der Gruppe dpp-X1, dpp-X2 und dpp-j-9 (Seq. ID 2-7, 15, 16, 23-29, 34-38, 44 und 49-51) verschiedene Längen und Sequenzen der Substratbindungsdomänen I und II hinsichtlich ihrer Spaltungseigenschaften an der Zielsequenz zu zeigen. Die Ergebnisse dieser Spaltungsexperimente sind in den Ausführungsbeispielen dargestellt.

In einer Ausführung sind die Substratbindungsdomänen I und II vollständig komplementär zu der speziellen Zielsequenz in der 5'-UTR Region des Virusgenoms. Um RNA in der 5'-UTR Region des Virusgenoms zu spalten, müssen die Substratbindungsdomänen I und II der erfindungsgemäßen DNAzyme jedoch nicht unbedingt vollständig komplementär sein. In vitro Untersuchungen zeigen, dass DNAzyme mit einer Substratbindungsdomäne I oder II die eine Homologie von 80%, 85%, 90% oder 95% aufweisen, ebenfalls an die Zielsequenz in der 5'-UTR Region des Virusgenoms binden und diese zu spalten vermögen.

### Modifikationen

Weiterhin wurde überraschenderweise gefunden, dass die erfindungsgemäßen DNAzyme ausgewählt aus der Gruppe dpp-X1, dpp-X2 und dpp-j-9 (Seq. ID 2-7, 15, 16, 23-29, 34-38, 44 und 49-51) durch Modifikationen gegenüber nukleolytischen Angriffen stabilisiert werden. Dies ist insbesondere für die Anwendung als medizinisches Mittel wichtig.

Die Modifikation bewirkt, dass die stabilisierten DNAzyme keine signifikante Verminderung der katalytischen Aktivität oder sogar eine verbesserte katalytische Aktivität gegenüber dem jeweiligen RNA-Substrat aufweisen.

Ein modifiziertes Nukleotid umfasst insbesondere eine chemische Modifizierung. Darunter versteht der Fachmann, dass das modifizierte Nukleotid durch Entfernen, Anfügen oder Ersetzen einzelner oder mehrerer Atome oder Atomgruppen im Vergleich zu natürlich vorkommenden Nukleotiden verändert ist. Die chemische Modifikation umfasst beispielsweise die Ribose (z.B. 2'-O-Methyl-Ribonukleotide, sog. "Locked Nucleic Acid" (LNA)-Ribonukleotide und invertiertes Thymidin), die Phosphor(di)esterbindung (z.B. Phosphorthioate, Phosphoramidate, Methylphosphonate und Peptidnukleotide) und/oder die Base (z.B. 7-Deazaguanosin, 5-Methylcytosin und Inosin).

In einer besonders bevorzugten Ausführungsform sind die erfindungsgemäßen DNAzyme an mindestens einem Nukleotid der Substratbindungsdomäne I und/oder II modifizierbar, insbesondere durch Phosphorthioat, invertiertes Thymidin, 2'-O-Methyl-Ribose oder LNA Ribonukleotide. Bei weiterhin bevorzugten DNAzymen ist ein Nukleotid oder sind mehrere Nukleotide der katalytischen Domäne modifiziert, insbesondere durch Phosphorthioat, invertiertes Thymidin, 2'-O-Methyl-Ribose oder LNA Ribonukleotide.

Eine bevorzugte Ausführung ist die Einführung einer 3'-3'-Inversion an einem Ende der erfindungsgemäßen DNAzyme. Der Begriff 3'-3'-Inversion bezeichnet eine kovalente Phosphatbindung zwischen den 3'-Kohlenstoffen des terminalen Nukleotids und des angrenzenden Nukleotids. Dieser Typ von Bindung steht im Gegensatz zu der normalen Phosphatbindung zwischen den 3' und 5' Kohlenstoffen von aufeinander folgenden Nukleotiden. Dementsprechend wird bevorzugt, dass das Nukleotid am 3'-Ende der an das 3'-Ende der katalytischen Domäne angrenzenden Substratbindungsdomäne invers ist. Zusätzlich zu den Inversionen können die DNAzyme modifizierte Nukleotide oder Nukleotid-Verbindungen enthalten. Modifizierte Nukleotide beinhalten z.B. N3'-P5'-Phosphoramidat Verbindungen, 2'-O-Methyl-Substitutionen und Peptid-Nukleinsäure-Verbindungen. Die Herstellung sämtlicher Modifikationen ist dem Fachmann auf diesem Gebiet geläufig und er findet Anleitungen zum Vorgehen im Stand der Technik.

In einer Ausführungsform ist die Modifikation in der katalytische Domäne lokalisiert. In einer weiteren Ausführungsform ist die Modifikation in der Substratbindungsdomäne I und/oder II lokalisiert. In einer weiteren Ausführungsform ist die Modifikation in der katalytischen Domäne und in der Substratbindungsdomäne I und/oder II lokalisiert.

### Verwendung als Arzneimittel

Mindestens eines der erfindungsgemäßen DNAzyme ausgewählt aus der Gruppe dpp-X1, dpp-X2 und dpp-j-9 (Seq. ID 2-7, 15, 16, 23-29, 34-38, 44 und 49-51) mit oder ohne Modifikationen ist Bestandteil eines medizinischen Mittels zur Prophylaxe und Behandlung von Virusinfektionen die durch Picornaviren insbesondere durch Rhinoviren verursacht bzw. hervorgerufen werden.

Das erfindungsgemäße Mittel umfasst alternativ einen pharmakologisch verträglichen Träger, Hilfsstoff und/oder Lösungsmittel.

Das erfindungsgemäße Mittel wird in Form von Tropfen, Mundspray, Nasenspray, Pillen, Tabletten, Filmtabletten, Schichttabletten, Zäpfchen, Gelen, Salben, Sirup, Inhalationspulvern, Granulaten, Suppositorien, Emulsionen, Dispersionen, Mikrokapseln, Kapseln, Puder oder Injektionslösungen hergestellt und verabreicht. Dies umfasst auch Formulierungen wie Schichttabletten zur kontrollierten und/oder kontinuierlichen Freisetzung sowie Mikroverkapselungen als spezielle Darreichungsform.

Das erfindungsgemäße Mittel umfasst Verkapselungen in Vesikel wie sie in der Dermatologie und Pharmazie zum Transport in die Haut bekannt sind z.B.: anionische oder kationische Liposomen, Niosome, Nanopartikel oder multilamellare Vesikel zur Penetration durch die Haut oder in die Zellen der Haut oder Haare. Das erfindungsgemäße Mittel ist unter anderem für die Inhalation oder die intravenöse, intraperitoneale, intramuskuläre, subkutane, mucokutane, orale, rektale, transdermale, topikale, bukkale, intradermale, intragastrale, intrakutane, intranasale, intrabuccale, perkutane oder sublinguale Verabreichung geeignet.

Als pharmakologisch verträgliche Träger werden beispielsweise Lactose, Stärke, Sorbitol, Sucrose, Cellulose, Magnesiumstearat, Dicalciumphosphat, Calciumsulfat, Talk, Mannitol, Ethylalcohol und dergleichen eingesetzt. Puder als auch Tabletten können zu 5 bis 95% aus einem derartigen Träger bestehen.

Ferner kann dem erfindungsgemäßen Mittel noch Sprengmittel, Farbstoffe, Geschmacksstoffe und/oder Bindemittel zugesetzt werden.

Flüssige Formulierungen umfassen Lösungen, Suspensionen, Sprays und Emulsionen, beispielsweise Injektionslösungen auf Wasserbasis oder Wasser-Propylenglycol-Basis für parenterale Injektionen.

Kapseln werden beispielsweise aus Methylcellulose, Polyvinylalkohole oder denaturierter Gelatine oder Stärke hergestellt.

Als Sprengmittel werden Stärke, Natrium-Carboxymethylstärke, natürliche und synthetische Gummis wie beispielsweise Johannisbrotkernmehl, Karaya, Guar, Tragacanth und Agar sowie Cellulosederivate wie Methylcellulose, NatriumCarboxymethylcellulose, microkristalline Cellulose sowie Alginate, Tonerden und Bentonite verwendet. Diese Bestandteile werden in Mengen von 2 bis 30 Gew.-% eingesetzt werden.

Als Bindemittel sind dem Fachmann Zucker, Stärke aus Korn, Reis oder Kartoffeln, natürliche Gummis wie Akaziengummi, Gelatine, Tragacanth, Alginsäure, Natriumalginat, Ammonium-Calcium-Alginat, Methylcellulose, Wachse, NatriumCarboxymethylcellulose, Hydroxypropyl-methylcellulose, Polyethylenglycol, Polyvinylpyrrolidon sowie anorganische Verbindungen wie Magnesium-Aluminum-Silicate bekannt, diese können dem erfindungsgemäßen Mittel zugesetzt werden. Die Bindemittel werden üblicherweise in Mengen von 1 bis 30 Gew.-% zugesetzt. Als Gleitmittel sind Borsäure und Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natriumoleat, Polyethylenglycol und Aminosäuren wie Leucin bekannt und werden eingesetzt. Derartige Gleitmittel werden üblicherweise in Mengen von 0,05 bis 15 Gew.-% verwendet.

Die Art der Dosierung des erfindungsgemäßen Mittels wird vom behandelnden Arzt entsprechend den klinischen Faktoren bestimmt. Es ist dem Fachmann bekannt, dass die Art der Dosierung von verschiedenen Faktoren wie z.B. Körpergröße, Gewicht, Körperoberfläche, Alter, Geschlecht oder der allgemeinen Gesundheit des Patienten abhängig ist, aber auch von dem speziell zu verabreichenden Mittel, der Dauer und Art der Verabreichung und von anderen Medikamenten, die möglicherweise parallel verabreicht werden.

Das medizinische Mittel ist zur Prophylaxe und Behandlung von Virusinfektionen die durch Picornaviren insbesondere durch Rhinoviren hervorgerufen werden, geeignet, insbesondere auch zur Prophylaxe und Behandlung gegen Rhinitis, Erkältung, Asthma, COPD, sowie zur Prophylaxe und Behandlung viraler Infektionen der oberen Atemwege.

Überraschenderweise wurde beobachtet, dass mit mindestens einem der erfindungsgemäßen DNAzyme ausgewählt aus der Gruppe dpp-X1, dpp-X2 und dpp-j-9 (Seq. ID 2-7, 15, 16, 23-29, 34-38, 44 und 49-51) auch eine generelle Prophylaxe gegen die Ausbildung und Manifestation von Asthma und COPD erreicht wird.

Die Kombination des mindestens einen erfindungsgemäßen DNAzyme ausgewählt aus der Gruppe dpp-X1, dpp-X2 und dpp-j-9 (Seq. ID 2-7, 15, 16, 23-29, 34-38, 44 und 49-51) mit mindestens einem Wirkstoff z.B. Analgetika und Antipyretika ist möglich.

Weiterhin ist die Kombination mit mindestens einem antiinflammatorischen Mittel, Immunmodulator, Antiasthmatikum und/oder Bronchodilatator möglich.

Ebenso ist die Kombination mit mindestens einem antiparasitären, antibakteriellen, antimykotischen und/oder antiviralen Wirkstoff möglich.

Alternativ ist die Kombination mit mindestens einem dermatologischen oder kosmetischen Wirkstoff möglich.

Das Mittel wird bevorzugt in Form von Lösungen und/oder Emulsionen in die Nase eines Patienten appliziert. Dazu kann es als Nasentropfen oder Nasenspray ausgebildet sein. Diese nasal applizierbaren Mittel wirken entweder zur Behandlung bzw. zur Vorbeugung an der Nase selbst oder führen zur Aufnahme des Mittels in den Blutkreislauf, damit sie die Wirkung an anderer Stelle im Körper entfalten.

Sie enthalten alternativ Hilfsstoffe zur Stabilisierung des Wirkstoffes bzw. zur Aufrechterhaltung eines bestimmten physiologisch akzeptablen pH-Wertes in der Nase. Für diesen Zweck kennt der Fachmann Phosphat- oder Phosphat/Citrat- oder Citrat-Puffer sowie Acetat-Puffer. Weitere Hilfsstoffe können Mittel zur Mund- und Rachendesinfektion oder Konservierungsmittel sein.

### Ausführungsbeispiele

### 1. Alignment, um eine möglichst optimale Zielsequenz im 5'-UTR Bereich des Virusgenoms von Picornaviren zu identifizieren

Die mittels Bioinformatik ausgewerteten Alignments sämtlicher verfügbarer Genomsequenzen von Picornaviren zeigen eine sehr große Homologie bzw. Sequenzidentität im 5'-UTR Bereich des Virusgenoms.

Die Sequenzen entstammen Quellen, die dem Fachmann bekannt sind z.B.
Enterovirus A: http://www.picornaviridae.com/enterovirus/ev-a/ev-a.htm plus http://www.picornaviridae.com/enterovirus/ev-a/ev-a seqs.htm
Enterovirus B: http://www.picornaviridae.com/enterovirus/ev-b/ev-b.htm
Enterovirus C: http://www.picornaviridae.com/enterovirus/ev-c/ev-c.htm
Enterovirus D: http://www.picornaviridae.com/enterovirus/ev-d/ev-d.htm
Enterovirus E: http://www.picornaviridae.com/enterovirus/ev-e/ev-e.htm
Enterovirus F: http://www.picornaviridae.com/enterovirus/ev-f/ev-f.htm
Enterovirus G: http://www.picornaviridae.com/enterovirus/ev-g/ev-g.htm
Enterovirus H: http://www.picornaviridae.com/enterovirus/ev-h/ev-h.htm
Enterovirus J: http://www.picornaviridae.com/enterovirus/ev-j/ev-j.htm

Dieser Bereich ist sehr konserviert innerhalb der Picornaviren und er weist eine geringe Mutationsrate auf. Genaue Auswertungen sämtlicher nach Mclntyre et al. J Gen Virol 2012 verfügbaren Genomsequenzen von Rhinoviren zeigen ebenfalls in diesem 5'-UTR Bereich eine sehr große Homologie bzw. Sequenzidentität. Dieser homologe 5'-UTR Bereich stellte somit eine gute Ausgangsposition dar, um DNAzyme bereitzustellen, die an diese Zielsequenz von möglichst viele Picornaviren und möglichst vielen Serotypen von Rhinoviren binden und diese spezifisch spalten.

Als Zielsequenz zur Bindung der erfindungsgemäßen DNAzyme wird der 5'-UTR Bereich mit der größten Homologie ermittelt.

Die GT (U) Spaltungsnukleotide sind fett dargestellt und unterstrichen: Seq ID 1 CTAGTTTGG**GTGT**CC**GT**GTTTC
Es wurde ermittelt, dass die erfindungsgemäßen DNAzyme ausgewählt aus der Gruppe dpp-X1, dpp-X2 und dpp-j-9 SeqlD 2-7, 15, 16, 23-29, 34-38, 44 und 49-51 spezifisch an der Zielsequenz von humanen Enteroviren (A-D), aber auch Schweine Enteroviren (G) und Affen Enteroviren (J) binden und diese spalten.

### 2. Plasmid Konstruktion

Die genomische RNA von allen verfügbaren Picornaviren wird nach einem dem Fachmann bekannten Verfahren oder mit einem kommerziell erhältlichen Kit z.B. dem QIAamp UltraSens Virus Kit nach Herstellerangaben isoliert und für cDNA Synthese verwendet. Die Synthese von cDNA erfolgt nach einem dem Fachmann bekannten Verfahren oder mit einem kommerziell erhältlichen Kit z.B. dem Omniscript Reverse Transcription Kit (Qiagen) und Zugabe eines RNaseOUT Ribonuclease Inhibitors (Invitrogen, Carlsbad, CA, USA) nach Herstellerangaben. Beispielhaft wird dies hier an genomischer RNA von Rhinovirus-Serotyp HRV-1b, -16 und -29 gezeigt.

Die cDNA für jedes Virus wird zur Amplifikation des spezifischen homologen 5'-UTR Bereich verwendet. Dazu werden PCR Reaktionen durchgeführt nach einem dem Fachmann bekannten Verfahren oder mit einem kommerziell erhältlichen Kit z.B. mit HotStarTaq Master Mix Kit (Qiagen) und sequenzspezifischen Primern. Die erhaltenen PCR Produkte werden über Gelelektrophorese (2% Agarose und 1xTBE TRIS-Borat-EDTA-Puffer) separiert und mit einem bekannten Verfahren oder mit einem kommerziell erhältlichen Kit z.B. dem Qiaquick Gel Extraction Kit (Qiagen) aus dem Gel extrahiert und bei -20°C gelagert. Die gereinigten PCR Produkte werden nach gängigen Verfahren in einen RNA-Expressionsvektor z.B. pGEM-T Easy Vector System II subkloniert. Das Ligationsprodukt wird in kompetente Zellen transformiert z.B. JM109 High Efficiency Competent cells. Dann erfolgt die Kultivierung in geeignetem Medium z.B. SOC Medium und die transformierten Bakterien werden auf Agarplatten ausplattiert z.B. Standard LB Agar mit Ampicillin und bis zur geeigneten Dichte kultiviert.

Positive Kulturen werden von den Platten in Mini-Preps in Standard LB Medium mit Ampicillin überführt und kultiviert. Plasmid DNA wird isoliert mit einem bekannten Verfahren oder mit einem kommerziell erhältlichen Kit z.B. dem QIAprep Spin Miniprep Kit (Qiagen). Die erste Verifikation der Klonierungeffizienz erfolgt mit E*coRI* (FastDigest *EcoRI,* Therma Fisher Scientific), Gelelektrophorese (1,5 % Agarose und 1xTBE TRIS-Borat-EDTA-Puffer), Sequenzierung mit Standardprimer SP6. Bakterien, die die gewünschte Sequenz enthalten werden in Maxi-Preps (Standard LB Medium, Ampicillin) kultiviert. Die Plasmid DNA Isolation erfolgt mit einem bekannten Verfahren oder mit einem kommerziell erhältlichen Kit z.B. dem HiSpeed Plasmid Maxi Kit (Qiagen). Gereinigte Plasmide werden bei -20°C gelagert. Alternativ erfolgt eine Kontrollsequenzierung mit Standard SP6 Primer.

### 3. RNA Expression (Synthese)

Die Plasmide werden mit bekannten Verfahren oder mit einem kommerziell erhältlichen Kit linearisiert unter Verwendung von Restriktionsenzymen z.B. *SpeI* oder *NcoI.* Die Proben werden mit Ethanol, EDTA und Ammoniumacetat präzipitiert und auf ihre Linearisierungseffizienz in der Gelelektrophorese (0,8 % Agarose und 1xTBE TRIS-Borat-EDTA-Puffer) getestet.

In vitro Transkription erfolgt mit einem bekannten Verfahren oder mit einem kommerziell erhältlichen Kit z.B. dem Ambion MEGAscript T7 Transkriptionskit oder dem Ambion MEGAscript SP6 Transkriptionskit gemäß Herstellerangaben. RNA wird gereinigt mit einem bekannten Verfahren oder mit einem kommerziell erhältlichen Kit z.B. dem RNeasy Mini Kit (*Qiagen*)*.* RNA Proben werden auf ihre Konzentration überprüft mit einem bekannten Verfahren oder mit einem kommerziell erhältlichen Kit z.B. dem NanoDrop 2000c (Thermo Fisher Scientific) und mit GelElektrophorese (2.5% Agarose, 1x TAE TRIS-Acetat-EDTA Puffer) analysiert. Die so erhaltenen RNA Moleküle entsprechen in ihrer Sequenz dem Insert umgeben von Vektorfragmenten zwischen dem T7 oder SP6 Polymerase Transkription Startstellen und 5' (genomischem) Ende vom Insert und zwischen 3' (genomischem) Ende vom Insert und *SpeI* oder *NcoI* Spaltungsstelle.

In allen Experimenten wird die Bindung der erfindungsgemäßen DNAzyme (Seq. ID 2-7, 15, 16, 23-29, 34-38, 44 und 49-51) am 5'-UTR Bereich folgender Typen von Picornaviren ermittelt:
A1, A2, A7, A8, A9, A10, A11, A12, A13, A15, A16, A18, A19, A20, A21, A22, A23, A24, A25, A28, A29, A30, A31, A32, A33, A34, A36, A38, A39, A40, A41, A43, A45, A46, A47, A49, A50, A51, A53, A54, A55, A56, A57, A58, A59, A60, A61, A62, A63, A64, A65, A66, A67, A68, A71, A73, A74, A75, A76, A77, A78, A80, A81, A82, A88, A89, A90, A94, A96, A100, A101, A102, A103, A104, A105, A106,
B3, B4, B5, B6, B14, B17, B26, B27, B35, B37, B42, B48, B52, B69, B70, B72, B79, B83, B84, B86, B91, B92, B93, B97, B99, B100, B101, B102, B103, B104, C1, C2, C3, C4, C5, C6, C7, C8, C9, C10, C11, C12, C15, C17, C22, C25, C26, C28, C32, C34, C35, C36, C38 C39, C40, C41, C42, C43, C45, C49, C51.

### 4. Bereitstellung der erfindungsgemäßen DNAzyme

Die Herstellung der erfindungsgemäßen DNAzyme (Seq. ID 2-7, 15, 16, 23-29, 34-38, 44 und 49-51) erfolgt nach einem dem Fachmann bekannten Syntheseverfahren.

### 5. Spaltungsassays zur Charakterisierung der erfindungsgemäßen DNAzyme an der Zielsequenz

Die Spaltungsassays beinhalten eine qualitative und quantitative Analyse in welchem Ausmaß die erfindungsgemäßen DNAzyme der (Seq. ID 2-7, 15, 16, 23-29, 34-38, 44 und 49-51) die Zielsequenz im 5'-UTR Bereich des Virusgenoms der Picornaviren insbesondere der Rhinoviren binden und spalten.

Die Spaltungsreaktion wird durchgeführt mit 1 µl Reaktionspuffer z.B. 500 mM TRIS, 1 µl 1 M NaCl, 1 µl 10 mM MgCl₂) und variablen Mengen an RNA und DNAzyme (zwischen 50 und 250 ng bzw. zwischen 10 und 30 pmol) und doppelt destilliertem Wasser (bis zu 10 µl Gesamtvolumen). In quantitativen Experimenten wird als Kontrolle eine Referenz RNA (z.B.: *GATA3* mRNA) zum Reaktionsansatz hinzugefügt. Die Reaktionsansätze werden bei 37°C für 60 min inkubiert, kommen dann auf Eis und werden durch Zugabe von Ambion Gel Loading Puffer II (Thermo Fisher Scientific) denaturiert, um die Reaktion zu stoppen.

Nach 10 min Inkubation bei 65°C werden die Reaktionsansätze über eine Gelelektrophorese (2.5% Agarose, 1x TAE Puffer) getrennt und graphisch z.B. im Fusion Fx7 System (PeqLab) dargestellt. Zur quantitativen Erfassung werden die Gelbilder anschließend noch durch Banden Dichtemessung z.B. mit dem LabImage 1D L340 Bio-Imaging ausgewertet. Für die Reduktion des Hintergrundes wird der "rolling ball mode" verwendet.

### 6. Verlängerung und Verkürzungen der Substratbindungsdomänen I und II

Die Erfinder haben in eigenen Forschungsarbeiten eine Serie spezieller DNAzyme mit spezifischen Substratbindungsdomänen I und II hergestellt und auf ihre Wirksamkeit an der speziellen Zielsequenz im 5'-UTR Bereich des Virusgenoms bei verschiedenen Serotypen von Rhinoviren und anderen Picornaviren analysiert. Die Wirksamkeit wird in Bindungs- und Spaltungsassays dargestellt.

Insbesondere weisen die DNAzyme ausgewählt aus der Gruppe dpp-X1, dpp-X2 und dpp-j-9 (Seq. ID 2-7, 15, 16, 23-29, 34-38, 44 und 49-51) unerwartet positive Eigenschaften auf hinsichtlich
- Bindung an Zielsequenz im 5'-UTR Bereich des Virusgenoms
- Starke enzymatische Spaltungsaktivität an Zielsequenz im 5'-UTR Bereich
- Starke Inaktivierung der Virus RNA bei verschiedenen Serotypen von Rhinoviren und anderen Picornaviren

Die Effizienz der Spaltung der erfindungsgemäßen DNAzyme an der Zielsequenz im 5'-UTR Bereich des Virusgenoms wird in einem Spaltungsassay mit RNA von verfügbaren Picornaviren z.B. den Rhinovirustypen HRV-1b, -16 und -29 gezeigt.

| DNAzym | | | Spaltung in Serotypen von Rhinoviren im 5'-UTR Bereich | | |
|---|---|---|---|---|---|
| Name | Substratbindungsdomäne **I** | Substratbindungsdomäne II | HRV 1B RNA Fragment | HRV 16 RNA Fragment | HRV 29 RNA Fragment |
| SeqlD 60 dpp-X1 | ACACGGACA | CCAAAGTAG | + | + | + |
| SeqID 61 dpp-X2 | AAACACGGA | ACCCAAAGT | + | + | + |
| SeqID 62 dpp-j-9 | AGTGAAACA | GGACACCCA | ++ | + | + |

Die katalytische Domäne weist eine Sequenz von GGCTAGCTACAACGA auf.

Es wurde ebenfalls gefunden, dass die Sequenzfolge GT der erfindungsgemäßen DNAzyme sehr bedeutend ist, da Modifikation mit einer Substitution zu GC keine Bindung an die Zielsequenz in der 5'-UTR Region des Virusgenoms mehr zeigten. Von den erfindungsgemäßen DNAzymen ausgewählt aus der Gruppe dpp-X1, dpp-X2 und dpp-j-9 (Seq. ID 2-7, 15, 16, 23-29, 34-38, 44 und 49-51) sowie von weiteren DNAzymen dieser Gruppen (Seq. ID 8-14, 17-22, 30-33, 39-43, 45-48 und 52-62) werden Längenvariationen in den Substratbindungsdomänen I bzw. II vorgenommen, die katalytische Domäne bleibt unverändert.

### a) DNAzym dpp-X1 zeigt eine gute Spaltungsaktivität an RNA von 97,76 % der verfügbaren Picornaviren z.B. den Rhinovirustypen HRV-1b, -16 und -29.

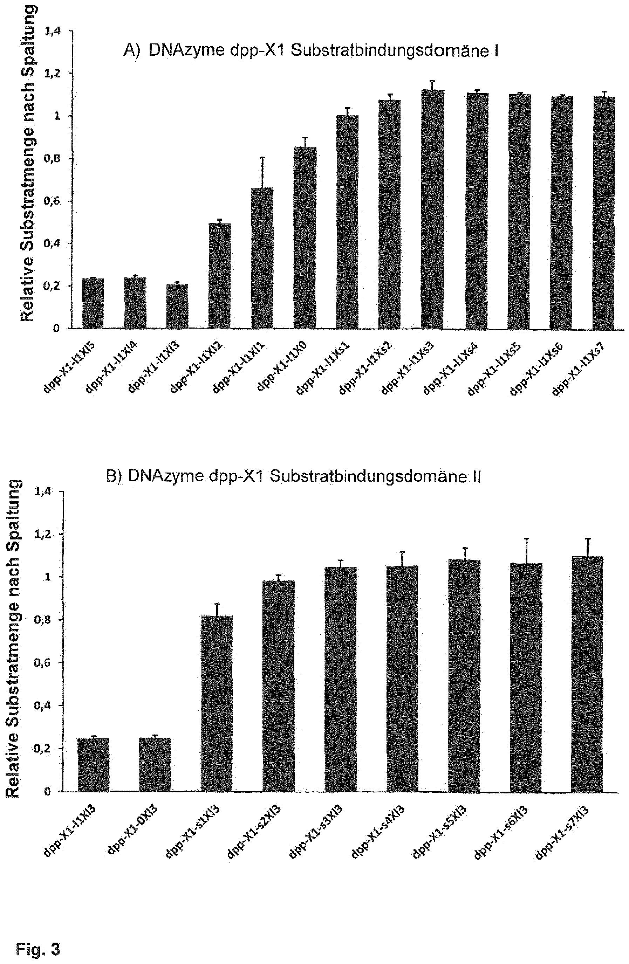

Es werden folgende Längenvarianten der Substratbindungsdomäne I vorgenommen, bei gleichbleibend 10 Nukleotiden in der Substratbindungsdomäne II und der katalytischen Domäne. Die Spaltungseigenschaften werden im Spaltungsassay getestet, Fig. 3 A:

| DNAzym | | | | |
|---|---|---|---|---|
| SeqID | Name | Substratbindungsdomäne I | katalytische Domäne | Substratbindungsdomäne II |
| 2 | dpp-X1-l1Xl5 | GTGAAACACGGACA | GGCTAGC TACAACGA | CCAAAGTAGT |
| 3 | dpp-X1-l1Xl4 | TGAAACACGGACA | GGCTAGC TACAACGA | CCAAAGTAGT |
| 4 | dpp-X1-l1Xl3 | GAAACACGGACA | GGCTAGC TACAACGA | CCAAAGTAGT |
| 5 | dpp-X1-l1Xl2 | AAACACGGACA | GGCTAGC TACAACGA | CCAAAGTAGT |
| 6 | dpp-X1-l1Xl1 | AACACGGACA | GGCTAGC TACAACGA | CCAAAGTAGT |
| 7 | dpp-X1-l1X0 | ACACGGACA | GGCTAGC TACAACGA | CCAAAGTAGT |
| 8 | dpp-X1-l1 Xs1 | CACGGACA | GGCTAGC TACAACGA | CCAAAGTAGT |
| 9 | dpp-X1-l1 Xs2 | ACGGACA | GGCTAGC TACAACGA | CCAAAGTAGT |
| 10 | dpp-X1-l1 Xs3 | CGGACA | GGCTAGC TACAACGA | CCAAAGTAGT |
| 11 | dpp-X1-l1 Xs4 | GGACA | GGCTAGC TACAACGA | CCAAAGTAGT |
| 12 | dpp-X1-l1 Xs5 | GACA | GGCTAGC TACAACGA | CCAAAGTAGT |
| 13 | dpp-X1-l1 Xs6 | ACA | GGCTAGC TACAACGA | CCAAAGTAGT |
| 14 | dpp-X1-l1 Xs7 | CA | GGCTAGC TACAACGA | CCAAAGTAGT |

Es werden folgende Längenvarianten der Substratbindungsdomäne II vorgenommen, bei gleichbleibend 12 Nukleotiden in der Substratbindungsdomäne I und auf ihre Spaltungseigenschaften im Spaltungsassay getestet, Fig. 3 B:

| DNAzym | | | | |
|---|---|---|---|---|
| SeqID | Name | Substratbindungsdomäne I | katalytische Domäne | Substratbindungsdomäne II |
| 4 | dpp-X1-l1Xl3 | GAAACACGGACA | GGCTAGCTA CAACGA | CCAAAGTAGT |
| 15 | dpp-X1-0Xl3 | GAAACACGGACA | GGCTAGCTA CAACGA | CCAAAGTAG |
| 16 | dpp-X1-s1Xl3 | GAAACACGGACA | GGCTAGCTA CAACGA | CCAAAGTA |
| 17 | dpp-X1-s2Xl3 | GAAACACGGACA | GGCTAGCTA CAACGA | CCAAAGT |
| 18 | dpp-X1-s3Xl3 | GAAACACGGACA | GGCTAGCTA CAACGA | CCAAAG |
| 19 | dpp-X1-s4Xl3 | GAAACACGGACA | GGCTAGCTA CAACGA | CCAAA |
| 20 | dpp-X1-s5Xl3 | GAAACACGGACA | GGCTAGCTA CAACGA | CCAA |
| 21 | dpp-X1-s6Xl3 | GAAACACGGACA | GGCTAGCTA CAACGA | CCA |
| 22 | dpp-X1-s7Xl3 | GAAACACGGACA | GGCTAGCTA CAACGA | CC |

Die Minimalsequenz für erfindungsgemäße DNAzyme der Gruppe dpp-X1 (Seq ID 2-7, 15 und 16) liegt bei mind. 8 Nukleotiden für die Substratbindungsdomäne I und mind. 8 Nukleotide für Substratbindungsdomäne II. Insbesondere bei Substratbindungsdomäne I CACGGACA und Substratbindungsdomäne II CCAAAGTA.

Die Länge der Substratbindungsdomänen I oder II kann auch länger als die angebenden Nukleotide sein. In einer Ausführung sind die Substratbindungsdomänen I und II gleich lang. In einer weiteren Ausführung sind die Substratbindungsdomänen I und II unterschiedlich lang.

### b) DNAzym dpp-X2 zeigt eine gute Spaltungsaktivität an RNA von 97,76 % der verfügbaren Picornaviren z.B. den Rhinovirustypen HRV-1b, -16 und -29.

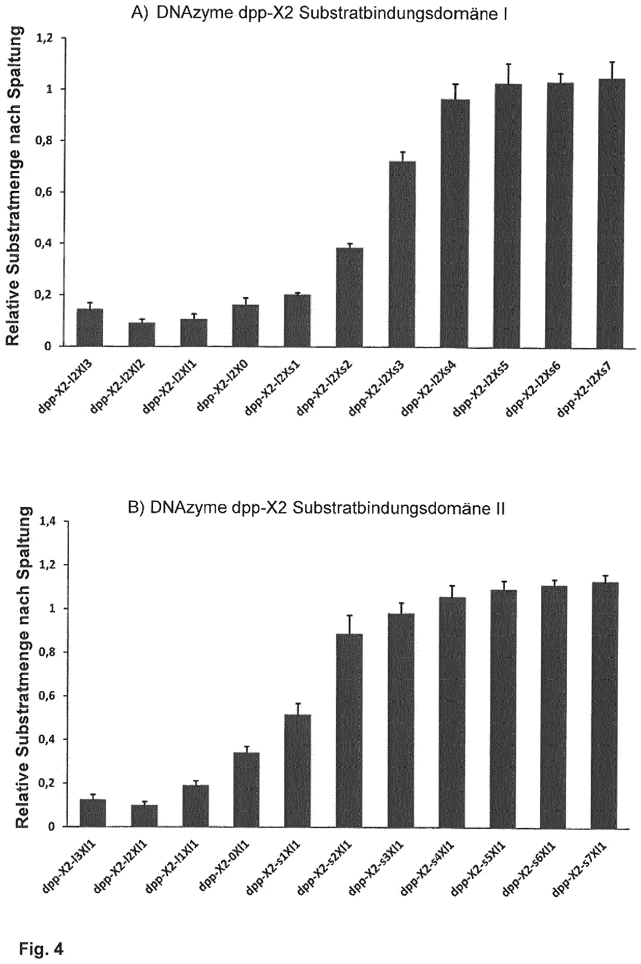

Es werden folgende Längenvarianten der Substratbindungsdomäne I vorgenommen, bei gleichbleibend 11 Nukleotiden in der Substratbindungsdomäne II und auf ihre Spaltungseigenschaften im Spaltungsassay getestet Fig.4 A:

| DNAzym | | | | |
|---|---|---|---|---|
| SeqID | Name | Substratbindungsdomäne I | katalytische Domäne | Substratbindungsdomäne II |
| 23 | dpp-X2-l2Xl3 | GTGAAACACGGA | GGCTAGCT ACAACGA | ACCCAAAGTAG |
| 24 | dpp-X2-l2Xl2 | TGAAACACGGA | GGCTAGCT ACAACGA | ACCCAAAGTAG |
| 25 | dpp-X2-l2Xl1 | GAAACACGGA | GGCTAGCT ACAACGA | ACCCAAAGTAG |
| 26 | dpp-X2-l2X0 | AAACACGGA | GGCTAGCT ACAACGA | ACCCAAAGTAG |
| 27 | dpp-X2-l2Xs1 | AACACGGA | GGCTAGCT ACAACGA | ACCCAAAGTAG |
| 28 | dpp-X2-l2Xs2 | ACACGGA | GGCTAGCT ACAACGA | ACCCAAAGTAG |
| 29 | dpp-X2-l2Xs3 | CACGGA | GGCTAGCT ACAACGA | ACCCAAAGTAG |
| 30 | dpp-X2-l2Xs4 | ACGGA | GGCTAGCT ACAACGA | ACCCAAAGTAG |
| 31 | dpp-X2-l2Xs5 | CGGA | GGCTAGCT ACAACGA | ACCCAAAGTAG |
| 32 | dpp-X2-l2Xs6 | GGA | GGCTAGCT ACAACGA | ACCCAAAGTAG |
| 33 | dpp-X2-l2Xs7 | GA | GGCTAGCT ACAACGA | ACCCAAAGTAG |

Es werden folgende Längenvarianten der Substratbindungsdomäne II vorgenommen, bei gleichbleibend 10 Nukleotiden in der Substratbindungsdomäne I und auf ihre Spaltungseigenschaften im Spaltungsassay getestet Fig.4 B:

| DNAzym | | | | |
|---|---|---|---|---|
| SeqID | Name | Substratbindungsdomäne I | katalytische Domäne | Substratbindungsdomäne II |
| 34 | dpp-X2-l3Xl1 | GAAACACGGA | GGCTAGCT ACAACGA | ACCCAAAGTAGT |
| 25 | dpp-X2-l2Xl1 | GAAACACGGA | GGCTAGCT ACAACGA | ACCCAAAGTAG |
| 35 | dpp-X2-l1Xl1 | GAAACACGGA | GGCTAGCT ACAACGA | ACCCAAAGTA |
| 36 | dpp-X2-0Xl1 | GAAACACGGA | GGCTAGCT ACAACGA | ACCCAAAGT |
| 37 | dpp-X2-s1Xl1 | GAAACACGGA | GGCTAGCT ACAACGA | ACCCAAAG |
| 38 | dpp-X2-s2Xl1 | GAAACACGGA | GGCTAGCT ACAACGA | ACCCAAA |
| 39 | dpp-X2-s3Xl1 | GAAACACGGA | GGCTAGCT ACAACGA | ACCCAA |
| 40 | dpp-X2-s4Xl1 | GAAACACGGA | GGCTAGCT ACAACGA | ACCCA |
| 41 | dpp-X2-s5Xl1 | GAAACACGGA | GGCTAGCT ACAACGA | ACCC |
| 42 | dpp-X2-s6Xl1 | GAAACACGGA | GGCTAGCT ACAACGA | ACC |
| 43 | dpp-X2-s7Xl1 | GAAACACGGA | GGCTAGCT ACAACGA | AC |

Die Minimalsequenz für erfindungsgemäße DNAzyme der Gruppe dpp-X2 (Seq ID 23-29 und 34-38) liegt bei mind. 6 Nukleotiden für die Substratbindungsdomäne I und mindestens 7 Nukleotide für Substratbindungsdomäne II. Insbesondere bei Substratbindungsdomäne I CACGGA und Substratbindungsdomäne II ACCCAAA. Die Länge der Substratbindungsdomänen I oder II kann auch länger als die angebenden Nukleotide sein. In einer Ausführung sind die Substratbindungsdomänen I und II gleich lang. In einer weiteren Ausführung sind die Substratbindungsdomänen I und II unterschiedlich lang.

### c) DNAzym dpp-j-9 zeigt eine Spaltungsaktivität an RNA von 97,76 % der verfügbaren Picornaviren z.B. den Rhinovirustypen HRV-1b, -16 und -29.

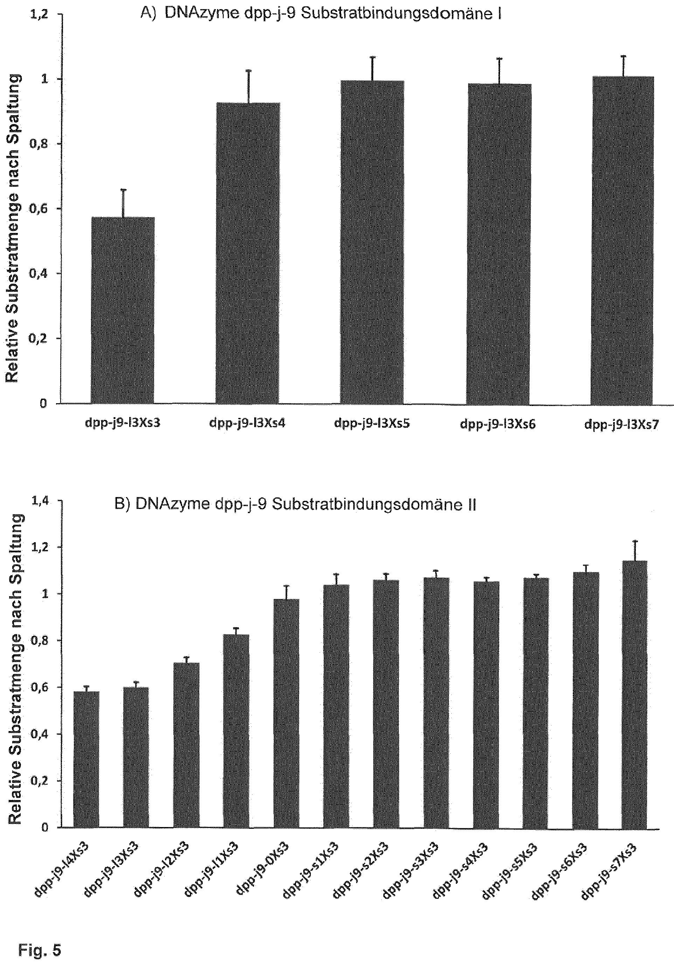

Es werden folgende Längenvarianten der Substratbindungsdomäne I vorgenommen, bei gleichbleibend 12 Nukleotiden in der Substratbindungsdomäne II und auf ihre Spaltungseigenschaften im Spaltungsassay getestet Fig.5 A:

| DNAzym | | | | |
|---|---|---|---|---|
| SeqID | Name | Substratbindungsdomäne I | katalytische Domäne | Substratbindungsdomäne II |
| 44 | dpp-j-9-l3Xs3 | GAAACA | GGCTAGCTA CAACGA | GGACACCCAAAG |
| 45 | dpp-j-9-l3Xs4 | AAACA | GGCTAGCTA CAACGA | GGACACCCAAAG |
| 46 | dpp-j-9-l3Xs5 | AACA | GGCTAGCTA | GGACACCCAAAG |
| | | | CAACGA | |
| 47 | dpp-j-9-l3Xs6 | ACA | GGCTAGCTA CAACGA | GGACACCCAAAG |
| 48 | dpp-j-9-l3Xs7 | CA | GGCTAGCTA CAACGA | GGACACCCAAAG |

Es werden folgende Längenvarianten der Substratbindungsdomäne II vorgenommen, bei gleichbleibend 6 Nukleotiden in der Substratbindungsdomäne I und auf ihre Spaltungseigenschaften im Spaltungsassay getestet Fig.5 B:

| DNAzym | | | | |
|---|---|---|---|---|
| SeqlD | Name | Substratbindungsdomäne I | katalytische Domäne | Substratbindungsdomäne II |
| 49 | dpp-j-9-l4Xs3 | GAAACA | GGCTAGCT ACAACGA | GGACACCCAAAGT |
| 44 | dpp-j-9-l3Xs3 | GAAACA | GGCTAGCT ACAACGA | GGACACCCAAAG |
| 50 | dpp-j-9-l2Xs3 | GAAACA | GGCTAGCT ACAACGA | GGACACCCAAA |
| 51 | dpp-j-9-l1 Xs3 | GAAACA | GGCTAGCT ACAACGA | GGACACCCAA |
| 52 | dpp-j-9-0Xs3 | GAAACA | GGCTAGCT ACAACGA | GGACACCCA |
| 53 | dpp-j-9-s1 Xs3 | GAAACA | GGCTAGCT ACAACGA | GGACACCC |
| 54 | dpp-j-9-s2Xs3 | GAAACA | GGCTAGCT ACAACGA | GGACACC |
| 55 | dpp-j-9-s3Xs3 | GAAACA | GGCTAGCT ACAACGA | GGACAC |
| 56 | dpp-j-9-s4Xs3 | GAAACA | GGCTAGCT ACAACGA | GGACA |
| 57 | dpp-j-9-s5Xs3 | GAAACA | GGCTAGCT ACAACGA | GGAC |
| 58 | dpp-j-9-s6Xs3 | GAAACA | GGCTAGCT ACAACGA | GGA |
| 59 | dpp-j-9-s7Xs3 | GAAACA | GGCTAGCT ACAACGA | GG |

Die Minimalsequenz für erfindungsgemäße DNAzyme der Gruppe dpp-j-9 (Seq ID 44 und 49-51) liegt bei mind. 6 Nukleotiden für die Substratbindungsdomäne I und mindestens 10 Nukleotide für Substratbindungsdomäne II.

Insbesondere bei Substratbindungsdomäne I GAAACA und Substratbindungsdomäne II GGACACCCAA. Die Länge der Substratbindungsdomänen I oder II kann auch länger als die angebenden Nukleotide sein. In einer Ausführung sind die Substratbindungsdomänen I und II gleich lang. In einer weiteren Ausführung sind die Substratbindungsdomänen I und II unterschiedlich lang.

### Abbildungslegenden und Bezugszeichenliste

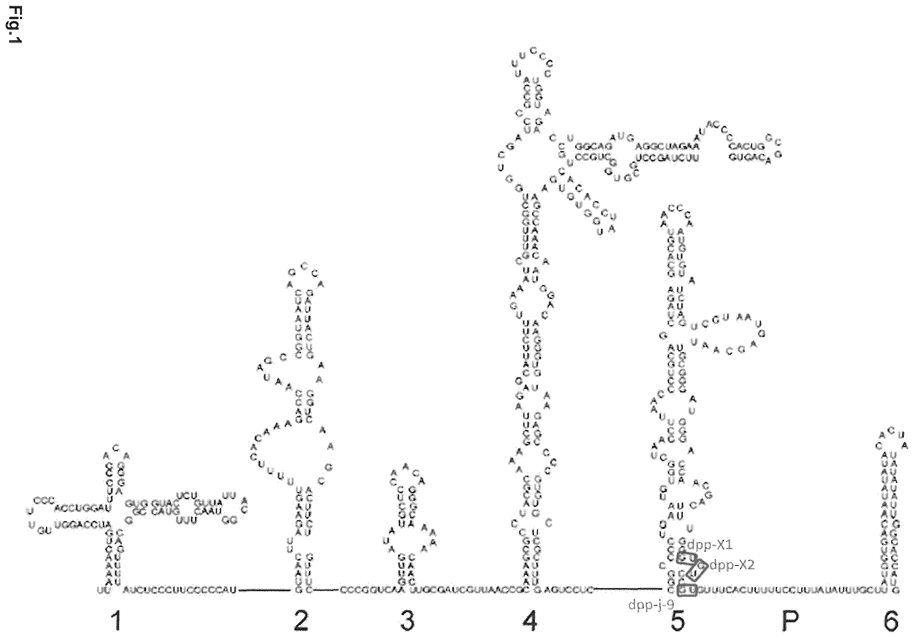

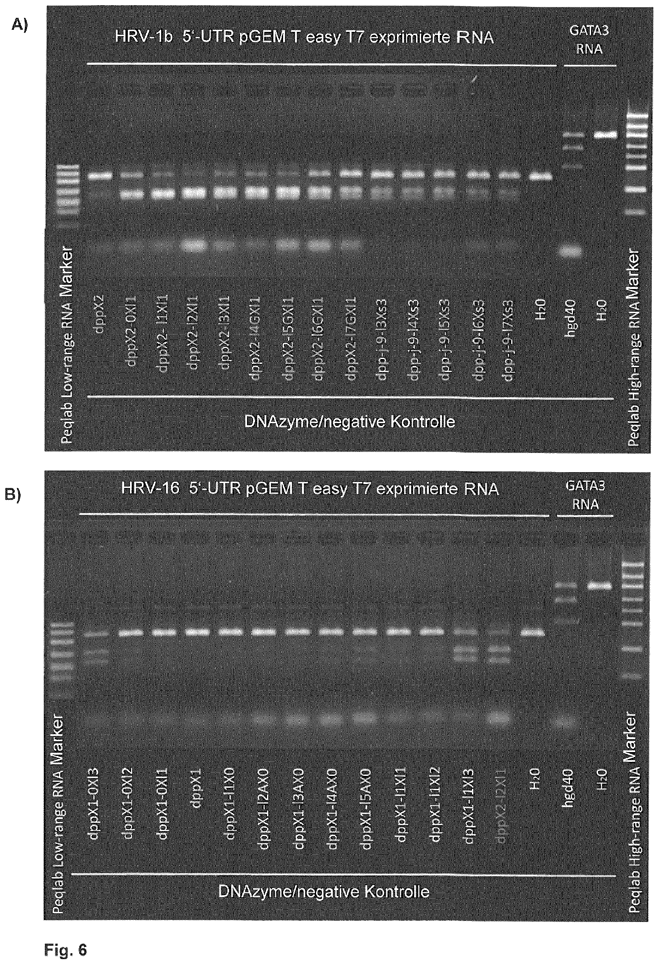

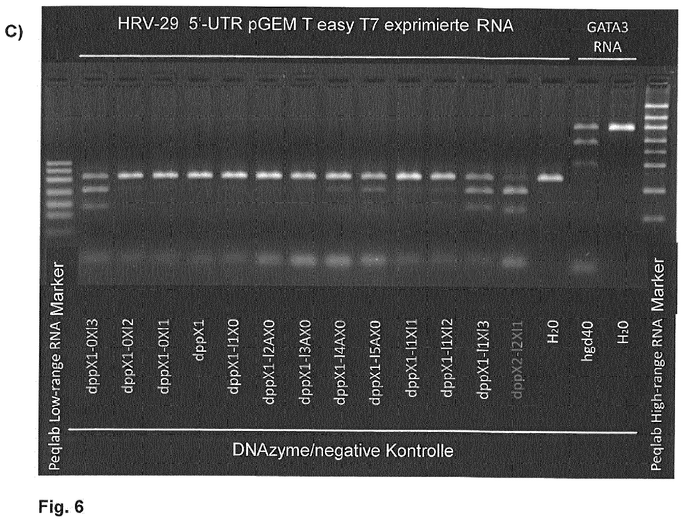

**Fig.1** zeigt die verschiedenen Serotypen die der Gruppe der humanen Rhinoviren nach aktueller Einteilung zugeordnet werden

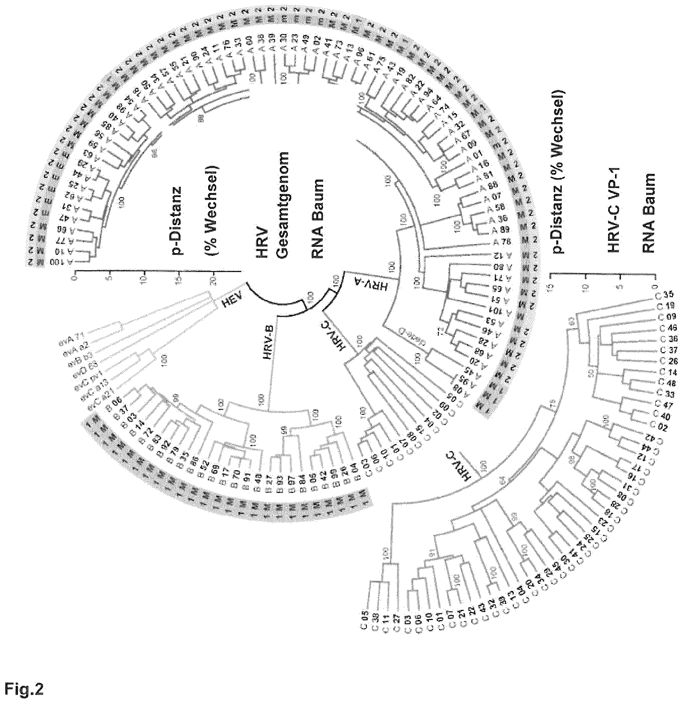

**Fig. 2** zeigt die Zielsequenz (Seq. ID 1) CTAGTTTGG**GTGT**CC**GT**GTTTC innerhalb des 5'-UTR Bereiches. Dieser Bereich weist eine sehr große Sequenzhomologie und geringe Mutationsrate innerhalb aller verfügbaren Virusgenome der Picornaviren und Rhinoviren auf.
   Dargestellt ist die Sekundärstruktur am Beispiel des humanen Rhinovirus 2 Virus mit sechs Stem-Loop Strukturen (Subdomänen 1-6) und dem Polypyrimidin-Trakt (P) zwischen Subdomäne 5 und 6. Eingezeichnet sind in Kästchen die Spaltungsregionen der erfindungsgemäßen DNAzyme dpp-X1, dpp-X2, dpp-j-9 (Seq. ID 2-7, 15, 16, 23-29, 34-38, 44 und 49-51)
**Fig. 3** zeigt die Spaltungsaktivität der Längenvarianten des DNAzyms dpp-X1 (Seq. ID 2-22) im Spaltungsassay
   A) Variationen der Substratbindedomäne I
   B) Variationen der Substratbindedomäne II
**Fig. 4** zeigt die Ergebnisse der Längenvarianten des DNAzyms dpp-X2 (Seq. ID 23-43) im Spaltungsassay
   A) Variationen der Substratbindedomäne I
   B) Variationen der Substratbindedomäne II
**Fig. 5** zeigt die Ergebnisse der Längenvarianten des DNAzyms dpp-j-9 (Seq. ID 44-59) im Spaltungsassay
   A) Variationen der Substratbindedomäne I
   B) Variationen der Substratbindedomäne II
**Fig. 6** zeigt in
   A) die Ergebnisse der Spaltungsexperimente der erfindungsgemäßen DNAzyme am HRV Serotyp 1b
   B) die Ergebnisse der Spaltungsexperimente der erfindungsgemäßen DNAzyme am HRV Serotyp 16
   C) die Ergebnisse der Spaltungsexperimente der erfindungsgemäßen DNAzyme am HRV Serotyp 29

## Patentansprüche

1. Mittel zur Prophylaxe und Behandlung von Virusinfektionen die durch Picornaviren verursacht werden, **dadurch gekennzeichnet, dass** es mindestens ein DNAzym vom Typ 10-23 ausgewählt aus der Gruppe bestehend aus Seq ID No. 2-7, 15, 16, 23-29, 34-38, 44 und 49-51 aufweist, wobei das DNAzym gemäß Seq ID Nr. 2-7, 15, 16 (dpp-X1) mindestens 8 Nukleotide für eine Substratbindungsdomäne I und mindestens 8 Nukleotide für eine Substratbindungsdomäne II aufweist, das DNAzym gemäß Seq ID Nr. 23-29, 34-38 (dpp-X2) mindestens 6 Nukleotide für die Substratbindungsdomäne I und mindestens 7 Nukleotide für Substratbindungsdomäne II aufweist und/oder das DNAzym gemäß Seq ID Nr. 44, 49-51 (dpp-j-9) mindestens 6 Nukleotide für die Substratbindungsdomäne I und mindestens 10 Nukleotide für Substratbindungsdomäne II aufweist.

2. Mittel zur Prophylaxe und Behandlung gemäß Anspruch 1, **dadurch gekennzeichnet, dass** das mindestens eine DNAzym ausgewählt ist aus der Gruppe bestehend aus Seq ID Nr. 2-7, 15, 16, 23-29, 34-38, 44 und 49-51.

3. Mittel zur Prophylaxe und Behandlung gemäß Anspruch 1 oder 2, **dadurch gekennzeichnet, dass** sich eine Zielsequenz in der 5'-UTR Region der Picornaviren befindet.

4. Mittel zur Prophylaxe und Behandlung gemäß einem der Ansprüche 1 - 3, **dadurch gekennzeichnet, dass** die Zielsequenz die 5'-UTR Region der Picornaviren gemäß SeqID1 ist.

5. Mittel zur Prophylaxe und Behandlung gemäß einem der vorangegangenen Ansprüche, **dadurch gekennzeichnet, dass** die Substratbindungsdomänen I und II unterschiedlich lang sind.

6. Mittel zur Prophylaxe und Behandlung gemäß einem der vorangegangenen Ansprüche, **dadurch gekennzeichnet, dass** die Substratbindungsdomänen I und II gleich lang sind.

7. Mittel zur Prophylaxe und Behandlung gemäß einem der vorangegangenen Ansprüche, **dadurch gekennzeichnet, dass** das mindestens eine DNAzym kombiniert werden kann mit mindestens einem antiinflammatorischen Mittel, Immunmodulator, Antiasthmatikum, Analgetikum, Antipyretikum und/oder Bronchodilatator.

8. Mittel zur Prophylaxe und Behandlung gemäß einem der vorangegangenen Ansprüche, **dadurch gekennzeichnet, dass** das mindestens eine DNAzym kombiniert werden kann mit mindestens einem antiparasitären, antibakteriellen, antimykotischen und/oder antiviralen Wirkstoff.

9. Mittel zur Prophylaxe und Behandlung gemäß einem der vorangegangenen Ansprüche, **dadurch gekennzeichnet, dass** es zur Prophylaxe von Rhinitis, Erkältung, Asthma, COPD und viraler Infektionen der oberen Atemwege geeignet ist.

10. Mittel zur Prophylaxe und Behandlung gemäß einem der vorangegangenen Ansprüche, **dadurch gekennzeichnet, dass** es zur Behandlung von Rhinitis, Erkältung, Asthma, COPD und viraler Infektionen der oberen Atemwege geeignet ist.

11. Mittel zur Prophylaxe und Behandlung gemäß einem der vorangegangenen Ansprüche, **dadurch gekennzeichnet, dass** das Mittel als Teil einer pharmazeutisch akzeptablen Komposition inhalativ, als Spray, Tropfen, oral oder in Tablettenform verabreicht wird.

## Claims

1. Agent for the prophylaxis and treatment of viral infections caused by picornaviruses, **characterised in that** it contains at least one DNAzyme of the type 10-23 selected from the group consisting of SEQ ID NO. 2-7, 15, 16, 23-29, 34-38, 44 and 49-51, wherein the DNAzyme according to SEQ ID NO. 2-7, 15, 16 (dpp-Xl) contains at least 8 nucleotides for a substrate binding domain I and at least 8 nucleotides for a substrate binding domain II, the DNAzyme according to SEQ ID NO. 23-29, 34-38 (dpp-X2) contains at least 6 nucleotides for the substrate binding domain I and at least 7 nucleotides for substrate binding domain II and/or the DNAzyme according to SEQ ID NO. 44, 49-51 (dpp-j-9) contains at least 6 nucleotides for the substrate binding domain I and at least 10 nucleotides for substrate binding domain II.

2. Agent for prophylaxis and treatment according to claim 1, **characterised in that** the at least one DNAzyme is selected from the group consisting of SEQ ID NO. 2-7, 15, 16, 23-29, 34-38, 44 and 49-51.

3. Agent for prophylaxis and treatment according to claim 1 or 2, **characterised in that** a target sequence is located in the 5'-UTR region of the picornaviruses.

4. Agent for prophylaxis and treatment according to any one of claims 1 to 3, **characterised in that** the target sequence is the 5'-UTR region of the picornaviruses according to SeqlD1.

5. Agent for prophylaxis and treatment according to any one of the preceding claims, **characterised in that** the substrate binding domains I and II are of different length.

6. Agent for prophylaxis and treatment according to any one of the preceding claims, **characterised in that** the substrate binding domains I and II are of equal length.

7. Agent for prophylaxis and treatment according to any one of the preceding claims, **characterised in that** the at least one DNAzyme can be combined with at least one anti-inflammatory agent, immunomodulator, antiasthmatic, analgesic, antipyretic and/or bronchodilator.

8. Agent for prophylaxis and treatment according to any one of the preceding claims, **characterised in that** the at least one DNAzyme can be combined with at least one antiparasitic, antibacterial, antimycotic and/or antiviral active substance.

9. Agent for prophylaxis and treatment according to any one of the preceding claims, **characterised in that** it is suitable for the prophylaxis of rhinitis, cold, asthma, COPD and viral infections of the upper respiratory tract.

10. Agent for prophylaxis and treatment according to any one of the preceding claims, **characterised in that** it is suitable for the treatment of rhinitis, cold, asthma, COPD and viral infections of the upper respiratory tract.

11. Agent for prophylaxis and treatment according to any one of the preceding claims, **characterised in that** the agent is administered as part of a pharmaceutically acceptable composition by inhalation, as a spray, as drops, orally or in tablet form.

## Revendications

1. Moyen pour la prophylaxie et le traitement d'infections virales provoquées par des Picornavirus, **caractérisé en ce qu'**il a au moins une DNAzyme des types 10-23, choisie dans le groupe consistant en SEQ ID NO: 2 à 7, 15, 16, 23 à 29, 34 à 38, 44 et 49 à 51, la DNAzyme selon SEQ ID NO: 2 à 7, 15, 16 (dpp-X1) a au moins 8 nucléotides pour le domaine de liaison au substrat I et au moins 8 nucléotides pour un domaine de liaison au substrat II, la DNAzyme selon SEQ ID NO: 23 à 29, 34 à 38 (dpp-X2) a au moins 6 nucléotides pour le domaine de liaison au substrat I et au moins 7 nucléotides pour le domaine de liaison au substrat II, et/ou la DNAzyme selon SEQ ID NO: 44, 49 à 51 (dpp-j-9) a au moins 6 nucléotides pour le domaine de liaison au substrat I et au moins 10 nucléotides pour le domaine de liaison au substrat II.

2. Moyen pour la prophylaxie et le traitement selon la revendication 1, **caractérisé en ce que** l'au moins une DNAzyme est choisie dans le groupe consistant en SEQ ID NO: 2 à 7, 15, 16, 23 à 29, 34 à 38, 44 et 49 à 51.

3. Moyen pour la prophylaxie et le traitement selon la revendication 1 ou 2, **caractérisé en ce qu'**une séquence cible se trouve dans la région 5'-UTR des Picornavirus.

4. Moyen pour la prophylaxie et le traitement selon l'une des revendications 1 à 3, **caractérisé en ce que** la séquence cible est la région 5'-UTR des Picornavirus selon SEQ ID1.

5. Moyen pour la prophylaxie et le traitement selon l'une des revendications précédentes, **caractérisé en ce que** les domaines de liaison au substrat I et II ont des longueurs différentes.

6. Moyen pour la prophylaxie et le traitement selon l'une des revendications précédentes, **caractérisé en ce que** les domaines de liaison au substrat I et II ont des longueurs identiques.

7. Moyen pour la prophylaxie et le traitement selon l'une des revendications précédentes, **caractérisé en ce que** l'au moins une DNAzyme peut être combinée à au moins un agent anti-inflammatoire, un immunomodulateur, un antiasthmatique, un analgésique, un antipyrétique et/ou un bronchodilatateur.

8. Moyen pour la prophylaxie et le traitement selon l'une des revendications précédentes, **caractérisé en ce que** l'au moins une DNAzyme peut être combinée à au moins un principe actif antiparasitaire, antibactérien, antimycotique et/ou antiviral.

9. Moyen pour la prophylaxie et le traitement selon l'une des revendications précédentes, **caractérisé en ce qu'**il convient à la prophylaxie de la rhinite, du rhume, de l'asthme, de la BPCO et des infections virales des voies respiratoires supérieures.

10. Moyen pour la prophylaxie et le traitement selon l'une des revendications précédentes, **caractérisé en ce qu'**il convient au traitement de la rhinite, du rhume, de l'asthme, de la BPCO et des infections virales des voies respiratoires supérieures.

11. Moyen pour la prophylaxie et le traitement selon l'une des revendications précédentes, **caractérisé en ce que** le moyen est administré en tant que partie d'une composition de qualité pharmaceutique, par inhalation sous forme de spray, de gouttes, par voie orale ou sous forme de comprimés.


### SEQUENCE LISTING

<110> Philipps-Universität Marburg
<120> Mittel zur Prophylaxe und Therapie von Virusinfektionen
<130> TM823
<140> EP
   <141> 2015-05-20
<160> 62
<170> Patent In version 3.5
<210> 1
   <211> 22
   <212> DNA
   <213> Picornavirus
<220>
   <221> Konsensus-Sequenz
   <222> (1)..(22)
   <223> Targetregion für Bindung und Spaltung der DNAzyme
<400> 1
   ctagtttggg tgtccgtgtt tc 22
<210> 2
   <211> 39
   <212> DNA
   <213> artificial
<220>
   <223> DNAzyme
<220>
   <221> dpp-X1-l1Xl5
   <222> (1)..(39)
<400> 2
   gtgaaacacg gacaggctag ctacaacgac caaagtagt 39
<210> 3
   <211> 38
   <212> DNA
   <213> artificial
<220>
   <223> DNAzyme
<220>
   <221> dpp-X1-l1Xl4
   <222> (1)..(38)
<400> 3
   tgaaacacgg acaggctagc tacaacgacc aaagtagt 38
<210> 4
   <211> 37
   <212> DNA
   <213> artificial
<220>
   <223> DNAzyme
<220>
   <221> dpp-X1-l1Xl3
   <222> (1)..(37)
<400> 4
   gaaacacgga caggctagct acaacgacca aagtagt 37
<210> 5
   <211> 36
   <212> DNA
   <213> artificial
<220>
   <223> DNAzyme
<220>
   <221> dpp-X1-l1Xl2
   <222> (1)..(36)
<400> 5
   aaacacggac aggctagcta caacgaccaa agtagt 36
<210> 6
   <211> 35
   <212> DNA
   <213> artificial
<220>
   <223> DNAzyme
<220>
   <221> dpp-X1-l1Xl1
   <222> (1)..(35)
<400> 6
   aacacggaca ggctagctac aacgaccaaa gtagt 35
<210> 7
   <211> 34
   <212> DNA
   <213> artificial
<220>
   <223> DNAzyme
<220>
   <221> dpp-X1-l1X0
   <222> (1)..(34)
<400> 7
   acacggacag gctagctaca acgaccaaag tagt 34
<210> 8
   <211> 33
   <212> DNA
   <213> artificial
<220>
   <223> DNAzyme
<220>
   <221> dpp-X1-l1Xs1
   <222> (1)..(33)
<400> 8
   cacggacagg ctagctacaa cgaccaaagt agt 33
<210> 9
   <211> 32
   <212> DNA
   <213> artificial
<220>
   <223> DNAzyme
<220>
   <221> dpp-X1-l1Xs2
   <222> (1)..(32)
<400> 9
   acggacaggc tagctacaac gaccaaagta gt 32
<210> 10
   <211> 31
   <212> DNA
   <213> artificial
<220>
   <223> DNAzyme
<220>
   <221> dpp-X1-l1Xs3
   <222> (1)..(31)
<400> 10
   cggacaggct agctacaacg accaaagtag t 31
<210> 11
   <211> 30
   <212> DNA
   <213> artificial
<220>
   <223> DNAzyme
<220>
   <221> dpp-X1-l1Xs4
   <222> (1)..(30)
<400> 11
   ggacaggcta gctacaacga ccaaagtagt 30
<210> 12
   <211> 29
   <212> DNA
   <213> artificial
<220>
   <223> DNAzyme
<220>
   <221> dpp-X1-l1Xs5
   <222> (1)..(29)
<400> 12
   gacaggctag ctacaacgac caaagtagt 29
<210> 13
   <211> 28
   <212> DNA
   <213> artificial
<220>
   <223> DNAzyme
<220>
   <221> dpp-X1-l1Xs6
   <222> (1)..(28)
<400> 13
   acaggctagc tacaacgacc aaagtagt 28
<210> 14
   <211> 27
   <212> DNA
   <213> artificial
<220>
   <223> DNAzyme
<220>
   <221> dpp-X1-l1Xs7
   <222> (1)..(27)
<400> 14
   caggctagct acaacgacca aagtagt 27
<210> 15
   <211> 36
   <212> DNA
   <213> artificial
<220>
   <223> DNAzyme
<220>
   <221> dpp-Xl-0Xl3
   <222> (1)..(36)
<400> 15
   gaaacacgga caggctagct acaacgacca aagtag 36
<210> 16
   <211> 35
   <212> DNA
   <213> artificial
<220>
   <223> DNAzyme
<220>
   <221> dpp-X1-s1Xl3
   <222> (1)..(35)
<400> 16
   gaaacacgga caggctagct acaacgacca aagta 35
<210> 17
   <211> 34
   <212> DNA
   <213> artificial
<220>
   <223> DNAzyme
<220>
   <221> dpp-X1-s2Xl3
   <222> (1)..(34)
<400> 17
   gaaacacgga caggctagct acaacgacca aagt 34
<210> 18
   <211> 33
   <212> DNA
   <213> artificial
<220>
   <223> DNAzyme
<220>
   <221> dpp-X1-s3Xl3
   <222> (1)..(33)
<400> 18
   gaaacacgga caggctagct acaacgacca aag 33
<210> 19
   <211> 32
   <212> DNA
   <213> artificial
<220>
   <223> DNAzyme
<220>
   <221> dpp-X1-s4Xl3
   <222> (1)..(32)
<400> 19
   gaaacacgga caggctagct acaacgacca aa 32
<210> 20
   <211> 31
   <212> DNA
   <213> artificial
<220>
   <223> DNAzyme
<220>
   <221> dpp-X1-s5Xl3
   <222> (1)..(31)
<400> 20
   gaaacacgga caggctagct acaacgacca a 31
<210> 21
   <211> 30
   <212> DNA
   <213> artificial
<220>
   <223> DNAzyme
<220>
   <221> dpp-X1-s6Xl3
   <222> (1)..(30)
<400> 21
   gaaacacgga caggctagct acaacgacca 30
<210> 22
   <211> 29
   <212> DNA
   <213> artificial
<220>
   <223> DNAzyme
<220>
   <221> dpp-X1-s7Xl3
   <222> (1)..(29)
<400> 22
   gaaacacgga caggctagct acaacgacc 29
<210> 23
   <211> 38
   <212> DNA
   <213> artificial
<220>
   <223> DNAzyme
<220>
   <221> dpp-X2-12Xl3
   <222> (1)..(38)
<400> 23
   gtgaaacacg gaggctagct acaacgaacc caaagtag 38
<210> 24
   <211> 37
   <212> DNA
   <213> artificial
<220>
   <223> DNAzyme
<220>
   <221> dpp-X2-l2Xl2
   <222> (1)..(37)
<400> 24
   tgaaacacgg aggctagcta caacgaaccc aaagtag 37
<210> 25
   <211> 36
   <212> DNA
   <213> artificial
<220>
   <223> DNAzyme
<220>
   <221> dpp-X2-l2Xl1
   <222> (1)..(36)
<400> 25
   gaaacacgga ggctagctac aacgaaccca aagtag 36
<210> 26
   <211> 35
   <212> DNA
   <213> artificial
<220>
   <223> DNAzyme
<220>
   <221> dpp-X2-l2X0
   <222> (1)..(35)
<400> 26
   aaacacggag gctagctaca acgaacccaa agtag 35
<210> 27
   <211> 34
   <212> DNA
   <213> artificial
<220>
   <223> DNAzyme
<220>
   <221> dpp-X2-l2Xs1
   <222> (1)..(34)
<400> 27
   aacacggagg ctagctacaa cgaacccaaa gtag 34
<210> 28
   <211> 33
   <212> DNA
   <213> artificial
<220>
   <223> DNAzyme
<220>
   <221> dpp-X2-l2Xs2
   <222> (1)..(33)
<400> 28
   acacggaggc tagctacaac gaacccaaag tag 33
<210> 29
   <211> 32
   <212> DNA
   <213> artificial
<220>
   <223> DNAzyme
<220>
   <221> dpp-X2-l2Xs3
   <222> (1)..(32)
<400> 29
   cacggaggct agctacaacg aacccaaagt ag 32
<210> 30
   <211> 31
   <212> DNA
   <213> artificial
<220>
   <223> DNAzyme
<220>
   <221> dpp-X2-l2Xs4
   <222> (1)..(31)
<400> 30
   acggaggcta gctacaacga acccaaagta g 31
<210> 31
   <211> 30
   <212> DNA
   <213> artificial
<220>
   <223> DNAzyme
<220>
   <221> dpp-X2-l2Xs5
   <222> (1)..(30)
<400> 31
   cggaggctag ctacaacgaa cccaaagtag 30
<210> 32
   <211> 29
   <212> DNA
   <213> artificial
<220>
   <223> DNAzyme
<220>
   <221> dpp-X2-l2Xs6
   <222> (1)..(29)
<400> 32
   ggaggctagc tacaacgaac ccaaagtag 29
<210> 33
   <211> 28
   <212> DNA
   <213> artificial
<220>
   <223> DNAzyme
<220>
   <221> dpp-X2-l2Xs7
   <222> (1)..(28)
<400> 33
   gaggctagct acaacgaacc caaagtag 28
<210> 34
   <211> 37
   <212> DNA
   <213> artificial
<220>
   <223> DNAzyme
<220>
   <221> dpp-X2-l3Xl1
   <222> (1)..(37)
<400> 34
   gaaacacgga ggctagctac aacgaaccca aagtagt 37
<210> 35
   <211> 35
   <212> DNA
   <213> artificial
<220>
   <223> DNAzyme
<220>
   <221> dpp-X2-l1Xl1
   <222> (1)..(35)
<400> 35
   gaaacacgga ggctagctac aacgaaccca aagta 35
<210> 36
   <211> 34
   <212> DNA
   <213> artificial
<220>
   <223> DNAzyme
<220>
   <221> dpp-X2-0Xl1
   <222> (1)..(34)
<400> 36
   gaaacacgga ggctagctac aacgaaccca aagt 34
<210> 37
   <211> 33
   <212> DNA
   <213> artificial
<220>
   <223> DNAzyme
<220>
   <221> dpp-X2-s1Xl1
   <222> (1)..(33)
<400> 37
   gaaacacgga ggctagctac aacgaaccca aag 33
<210> 38
   <211> 32
   <212> DNA
   <213> artificial
<220>
   <223> DNAzyme
<220>
   <221> dpp-X2-s2Xl1
   <222> (1)..(32)
<400> 38
   gaaacacgga ggctagctac aacgaaccca aa 32
<210> 39
   <211> 31
   <212> DNA
   <213> artificial
<220>
   <223> DNAzyme
<220>
   <221> dpp-X2-s3Xl1
   <222> (1)..(31)
<400> 39
   gaaacacgga ggctagctac aacgaaccca a 31
<210> 40
   <211> 30
   <212> DNA
   <213> artificial
<220>
   <223> DNAzyme
<220>
   <221> dpp-X2-s4Xl1
   <222> (1)..(30)
<400> 40
   gaaacacgga ggctagctac aacgaaccca 30
<210> 41
   <211> 29
   <212> DNA
   <213> artificial
<220>
   <223> DNAzyme
<220>
   <221> dpp-X2-s5Xl1
   <222> (1)..(29)
<400> 41
   gaaacacgga ggctagctac aacgaaccc 29
<210> 42
   <211> 28
   <212> DNA
   <213> artificial
<220>
   <223> DNAzyme
<220>
   <221> dpp-X2-s6Xl1
   <222> (1)..(28)
<400> 42
   gaaacacgga ggctagctac aacgaacc 28
<210> 43
   <211> 27
   <212> DNA
   <213> artificial
<220>
   <223> DNAzyme
<220>
   <221> dpp-X2-s7Xl1
   <222> (1)..(27)
<400> 43
   gaaacacgga ggctagctac aacgaac 27
<210> 44
   <211> 33
   <212> DNA
   <213> artificial
<220>
   <223> DNAzyme
<220>
   <221> dpp-j-9-l3Xs3
   <222> (1)..(33)
<400> 44
   gaaacaggct agctacaacg aggacaccca aag 33
<210> 45
   <211> 32
   <212> DNA
   <213> artificial
<220>
   <223> DNAzyme
<220>
   <221> dpp-j-9-l3Xs4
   <222> (1)..(32)
<400> 45
   aaacaggcta gctacaacga ggacacccaa ag 32
<210> 46
   <211> 31
   <212> DNA
   <213> artificial
<220>
   <223> DNAzyme
<220>
   <221> dpp-j-9-l3Xs5
   <222> (1)..(31)
<400> 46
   aacaggctag ctacaacgag gacacccaaa g 31
<210> 47
   <211> 30
   <212> DNA
   <213> artificial
<220>
   <223> DNAzyme
<220>
   <221> dpp-j-9-l3Xs6
   <222> (1)..(30)
<400> 47
   acaggctagc tacaacgagg acacccaaag 30
<210> 48
   <211> 29
   <212> DNA
   <213> artificial
<220>
   <223> DNAzyme
<220>
   <221> dpp-j-9-l3Xs7
   <222> (1)..(29)
<400> 48
   caggctagct acaacgagga cacccaaag 29
<210> 49
   <211> 34
   <212> DNA
   <213> artificial
<220>
   <223> DNAzyme
<220>
   <221> dpp-j-9-l4Xs3
   <222> (1)..(34)
<400> 49
   gaaacaggct agctacaacg aggacaccca aagt 34
<210> 50
   <211> 32
   <212> DNA
   <213> artificial
<220>
   <223> DNAzyme
<220>
   <221> dpp-j-9-l2Xs3
   <222> (1)..(32)
<400> 50
   gaaacaggct agctacaacg aggacaccca aa 32
<210> 51
   <211> 31
   <212> DNA
   <213> artificial
<220>
   <223> DNAzyme
<220>
   <221> dpp-j-9-l1Xs3
   <222> (1)..(31)
<400> 51
   gaaacaggct agctacaacg aggacaccca a 31
<210> 52
   <211> 30
   <212> DNA
   <213> artificial
<220>
   <223> DNAzyme
<220>
   <221> dpp-j-9-0Xs3
   <222> (1)..(30)
<400> 52
   gaaacaggct agctacaacg aggacaccca 30
<210> 53
   <211> 29
   <212> DNA
   <213> artificial
<220>
   <223> DNAzyme
<220>
   <221> dpp-j-9-s1Xs3
   <222> (1)..(29)
<400> 53
   gaaacaggct agctacaacg aggacaccc 29
<210> 54
   <211> 28
   <212> DNA
   <213> artificial
<220>
   <223> DNAzyme
<220>
   <221> dpp-j-9-s2Xs3
   <222> (1)..(28)
<400> 54
   gaaacaggct agctacaacg aggacacc 28
<210> 55
   <211> 27
   <212> DNA
   <213> artificial
<220>
   <223> DNAzyme
<220>
   <221> dpp-j-9-s3Xs3
   <222> (1)..(27)
<400> 55
   gaaacaggct agctacaacg aggacac 27
<210> 56
   <211> 27
   <212> DNA
   <213> artificial
<220>
   <223> DNAzyme
<220>
   <221> dpp-j-9-s4Xs3
   <222> (1)..(27)
<400> 56
   gaaacaggct agctacaacg aggacac 27
<210> 57
   <211> 26
   <212> DNA
   <213> artificial
<220>
   <223> DNAzyme
<220>
   <221> dpp-j-9-s5Xs3
   <222> (1)..(26)
<400> 57
   gaaacaggct agctacaacg aggaca 26
<210> 58
   <211> 24
   <212> DNA
   <213> artificial
<220>
   <223> DNAzyme
<220>
   <221> dpp-j-9-s6Xs3
   <222> (1)..(24)
<400> 58
   gaaacaggct agctacaacg agga 24
<210> 59
   <211> 23
   <212> DNA
   <213> artificial
<220>
   <223> DNAzyme
<220>
   <221> dpp-j-9-s7Xs3
   <222> (1)..(23)
<400> 59
   gaaacaggct agctacaacg agg 23
<210> 60
   <211> 33
   <212> DNA
   <213> artificial
<220>
   <223> DNAzyme
<220>
   <221> dpp-X1
   <222> (1)..(33)
<400> 60
   acacggacag gctagctaca acgaccaaag tag 33
<210> 61
   <211> 33
   <212> DNA
   <213> artificial
<220>
   <223> DNAzyme
<220>
   <221> dpp-X2
   <222> (1)..(33)
<400> 61
   aaacacggag gctagctaca acgaacccaa agt 33
<210> 62
   <211> 33
   <212> DNA
   <213> artificial
<220>
   <223> DNAzyme
<220>
   <221> dpp-j-9
   <222> (1)..(33)
<400> 62
   agtgaaacag gctagctaca acgaggacac cca 33